# EUROPEAN PATENT APPLICATION

(11) **EP 2 062 580 A1**
(43) Date of publication of application: **27.05.2009**
(21) Application number: 09152218.5
(22) Date of filing: 17.04.2004
(51) Int. Cl.: A61K 31/519, A61P 7/02

(54) **Use of dipyridamole or mopidamole for treatment and prevention of thromboembolic diseases and disorders caused by excessive formation of thrombin and/or by elevated expression of trombin receptors**

(30) Priority: 24.04.2003 US 465168 P
(62) Divisional of application: 04728114.2
(71) Applicant: Boehringer Ingelheim International GmbH, 55216 Ingelheim am Rhein (DE)
(72) Inventor: Eisert, Wolfgang, 30175 Hannover (DE); Serebruany, Victor, Ellicott City, MD 21042 (US)
(74) Representative: Hammann, Heinz

(57) **Abstract**

A method of treatment of the human or non-human animal body for treating or preventing disorders caused by elevated thrombin or elevated thrombin receptor expression is disclosed, for example thromboembolic disease vascular syndromes, or proliferative diseases, which method comprises administering to a human or non-human animal body in need of such treatment an effective amount of a pharmaceutical composition containing dipyridamole, mopidamole or a pharmaceutically acceptable salt thereof, corresponding pharmaceutical compositions as well as the use of dipyridamole or mopidamole for the manufacture of these pharmaceutical compositions.

## Description

### Field of the Invention

This invention relates to a method of treating and preventing thromboembolic diseases using dipyridamole or mopidamole as active principle, and the use of dipyridamole or mopidamole in combination with other compounds with the potential of amplifying the antithrombotic effect, corresponding pharmaceutical compositions as well as the manufacture of corresponding pharmaceutical compositions.

### Background of the Invention

Dipyridamole {2,6-bis(diethanolamino)-4,8-dipiperidino-pyrimido[5,4-d]pyrimidine}, closely related substituted pyrimido-pyrimidines and their preparation have been described in e.g. U.S.Patent 3,031,450. Further related substituted pyrimido-pyrimidines and their preparation have been described in e.g. GB 1,051,218, inter alia the compound mopidamol {2,8-bis(diethanolamino)-4--piperidinopyrimido[5,4-d]pyrimidine}. Dipyridamole was introduced as a *coronary vasodilator* in the early 1960s. It is also well known having *platelet aggregation inhibitor properties* due to the inhibition of adenosine uptake. Subsequently, dipyridamole was shown to reduce thrombus formation in a study of arterial circulation of the brain in a rabbit model. These investigations led to its use as an *antithrombotic agent;* it soon became the therapy of choice for such applications as stroke prevention, maintaining the patency of coronary bypass and valve-replacement, as well as for treatment prior to coronary angioplasty.

Furthermore, the European Stroke Prevention Study 2 (ESPS-2; J Neurol Sci. 1996; 143: 1-13; Neurology 1998; 51: 17-19) proved that treatment by dipyridamole alone was as effective as low-dose aspirin in the reduction of stroke risk, and combination therapy with dipyridamole and aspirin was more than twice as effective as aspirin alone.

Dipyridamole appears to inhibit thrombosis through multiple mechanisms. Early studies showed that it inhibits the uptake of adenosine, which was found to be a potent endogenous anti-thrombotic compound. Dipyridamole was also shown to inhibit cyclic AMP phosphodiesterase, thereby increasing intracellular c-AMP.

By laboratory models reflecting the complex physiology of the blood vessel it could be shown that the vasculature is not a passive conduit, but interacts profoundly with the blood through an intricate system of checks and balances to protect its integrity after vascular accident. Therefore the endothelium produces prostacyclin, a potent inhibitor of aggregation. The normal endothelium is not thrombogenic and prevents the attachment of platelets. Various stimulants precipitate the release of endothelium-derived relaxing factor (EDRF), which inhibits platelet adhesion and aggregation. At the same time, intracellular increase in cGMP was shown to be responsible for relaxation of smooth muscle cells following administration of nitro compounds. Thus the endothelium can inhibit thrombus formation by two separate mechanisms, one mediated by prostacyclin and c-AMP, and the other by EDRF and c-GMP. Dipyridamole appears to enhance both of these antithrombotic mechanisms of the vessel wall, in addition to its adenosine-sparing effects. It stimulates prostacyclin production by increasing intracellular levels of cAMP, and it enhances the strongly anti-thrombotic nitric oxide system by increasing cGMP.

Dipyridamole also has *antioxidant properties* (Free Radic. Biol. Med. 1995; 18: 239-247) that may contribute to its antithrombotic effect. When oxidized, low density lipoproteins become recognized by the scavenger receptor on macrophages, which is assumed to be the necessary step in the development of atherosclerosis (Ann. Rev. Med. 1992; 43: 219-25).

The inhibition of free radical formation by dipyridamole has been found to inhibit fibrinogenesis in experimental liver fibrosis (Hepatology 1996; 24: 855-864) and to suppress oxygen radicals and proteinuria in experimental animals with amino-nucleoside nephropathy (Eur. J. Clin. invest. 1998; 28: 877-883; Renal Physiol. 1984; 7: 218-226). Inhibition of lipid peroxidation also has been observed in human nonneoplastic lung tissue (Gen. Pharmacol. 1996; 27: 855-859).

Mopidamole is known to possess antithrombotic and additionally antimetastatic properties.

In WO 01/30353 is disclosed that fibrin-dependent microcirculation disorders can be treated by dipyridamole, for example microcirculation disorders caused by metabolic diseases, inflammatory reactions or autoimmune diseases, furthermore peripheral microcirculation disorders or microcirculation disorders associated with increased cell fragmentation.

Furthermore, WO 02/085331 discloses that NO-dependent microcirculation disorders can be treated by dipyridamole, due to the activity as free radical scavenger.

WO 02/34248 discloses a method for increasing tissue perfusion with blood by coadministration of an agent that increases cGMP synthesis and an agent that inhibits cGMP degradation in the cells of the blood vessel walls or in blood cells, e.g. by coadministration of a statin and dipyridamole.

Thrombin is one of the main triggers of thromboembolic disorders. Thrombin is formed within the clotting cascade by clotting factor V and X from its precursor Pro-Thrombin. Thrombin besides of its fibrin forming capacity activates platelets directly by binding to Thrombin receptors on the surface of the platelet as well as other cells relevant to the process of thrombin formation. Thrombin has also been described to react with thrombin receptors on the surface of vessel wall cells, stimmulating proliferation and migration of vessel wall cells. So far, direct inhibition of Thrombin via synthetic Thrombin Inhibitors or indirect inhibition by blocking clotting factor X or any combination of factors in the clotting cascade has been the method of choice to block either thromboembolic events or to block vessel wall thrombosis or restenosis. Furthermore embolic lodging of tumor metastasis has been connected with clotting activities.

The effects of thrombin are most obvious in areas of slow blood flow such as in low flow venous systems or locally circulating flow such as in vortices behind drastic lumen narrowing or in certain parts of the heart ventricle where wall motion is irregular leading to low or no flow in that part of the atrium or the ventricle. Those conditions are conventionally treated by inhibitors of the doting cascade or direct thrombin inhibitors or Thrombin receptor antagonists, such as unfractionated heparin, low molecular weight heparin, Hirulog or recently developed polyglycans.

### Brief Summary of the Invention

It has now surprisingly been found that dipyridamole and/or mopidamole alone and in combination with other compounds/drugs reducing the incidence of cardiovascular and cerebrovascular events reduce expression of PAR-1 Receptor (Thrombin Receptor) thus providing an approach for a method of treatment and/or prevention of thromboembolic and vascular disorders.

The finding that dipyridamole as well as mopidamole downregulate PAR-1 Receptor expression presumably as a result of intracellular cGMP elevation thus contributing to stabilize intracellular mechanisms provides a rationale also for combination treatment together with other antithrombotic agents, such as platelet aggregation inhibitors, e.g. acetylsalicalic acid (ASA), clopidogrel or ticlopidine or the pharmaceutically acceptable salts thereof, fibrinogen receptor antagonists (Abciximab, RDGS-peptides, synthetic i.v. or oral fibrinogen antagonists, e.g. fradafiban, lefradafiban or pharmaceutically acceptable salts thereof), heparin and heparinoids or antithrombins, or for combination treatment using additional cardiovascular therapies such as treatment with ACE inhibitors, Angiotensin II antagonists, Ca-antagonists or lipid-lowering agents such as the statins. It has been reported that statins, independent from their lipid-lowering activity, increase levels of nitric oxide (NO) well known to also stabilize cells by elevating intracellular cGMP levels. providing a rationale for a preferred combination of dipyridamole with a statin in the treatment of Thromboembolic disorders (J. Vasc. Surg. 2002, 36(1); 158-63).

In preventing processes leading to restenosis by activating thrombin receptors combination of Dipyridamole as well as Mopidamole with conventional antiproliferative agents such as TNF-alpha or others is preferred to further enhance the beneficial effect.

ASA inhibits aggregation through direct effects on the platelet, in more detail, by irreversibly acetylating platelet cyclooxygenase, thus inhibiting the production of thromboxane, which is strongly thrombotic. In high doses, however, aspirin crosses over into endothelial cells (N. Eng. J. Med. 1984; 311: 1206-1211), where it interrupts the production of prostacyclin, a potent natural inhibitor of platelet aggregation and by-product of the "arachidonic cascade" (N. Engl. J. Med. 1979; 300: 1142-1147). These observations led to the concept of low-dose antiplatelet therapy with ASA to maximize inhibition of thromboxane while minimizing the loss of prostacyclin (Lancet 1981; 1: 969-971). In combination with dipyridamole according to the invention also the low-dose ASA concept is preferred.

Viewed from one aspect the present invention provides a method of treatment of the human or non-human animal body, preferably mammalian body, for treating and/or preventing thromboembolic disorders or medical conditions, accompanied or characterized by global or local elevation of Thrombin in the plasma or localized elevation of Thrombin at a site of low blood flow or other conditions to increase thrombin formation, said method comprising administering to said body an effective amount of a pharmaceutical composition comprising an active ingredient selected from the group of dipyridamole, mopidamole and the pharmaceutically acceptable salts thereof, optionally in combination with one or more other antithrombotic agents, ACE inhibitors, Angiotensin II antagonists, Ca-antagonists or lipid-lowering agents such as statins.

Viewed from a different aspect the present invention provides the use of an active ingredient selected from the group of dipyridamole, mopidamole and the pharmaceutically acceptable salts thereof, optionally in combination with one or more other antithrombotic agents, ACE inhibitors, Angiotensin II antagonists, Ca-antagonists or lipid-lowering agents, for the manufacture of a pharmaceutical composition for the treatment of the human or non-human animal body, preferably mammalian body, for treating and/or preventing thromboembolic disorders or medical conditions accompanied or characterized by elevated or local thrombin plasma levels as well as increased expression of thrombin receptors.

Viewed from a further aspect the present invention provides a pharmaceutical composition comprising a pyrimido-pyrimidine selected from dipyridamole, mopidamole and the pharmaceutically acceptable salts thereof, and one or more drugs selected from other antithrombotic agents, ACE inhibitors, Angiotensin II antagonists, Ca-antagonists and lipid-lowering agents, optionally together with one or more excipients or carriers.

### Detailed Description of the Invention

The invention provides a new approach for the treatment and/or prevention of Thrombo-embolic disorders or medical conditions accompanied or characterized by elevated thrombin levels or increased expression of thrombin receptors, said method comprising administering to said body an effective amount of a pharmaceutical composition comprising an active ingredient selected from the group of dipyridamole, mopidamole and the pharmaceutically acceptable salts thereof, optionally in combination with one or more other antithrombotic agents, ACE inhibitors, Angiotensin II antagonists, Ca-antagonists or lipid-lowering agents particularly statins. Antithrombotic agents are meant to comprise particularly inhibitors of thrombin formation and thrombin antagonists, e.g. heparin, heparinoids, warfarin, the compound (A): 1-methyl-2-[N-(4-amidinophenyl)-aminomethyl]-benzimidazol-5-yl-carbox-ylic acid-N-(2-pyridyl)-N-(2-hydroxycarbonylethyl)-amide, described in WO 98/37075 having the structure 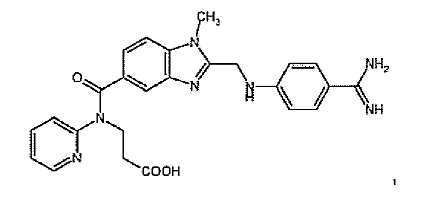 and the prodrugs thereof, such as compound (B): dabigatran etexilate (1-methyl-2-[N-[4-(N-n-hexyloxycarbonylamidino)phenyl]-aminomethyl]-benzimidazol-5-yl-carboxylic acid-N-(2-pyridyl)-N-(2-ethoxycarbonylethyl)-amide), also described in WO 98/37075 having the structure 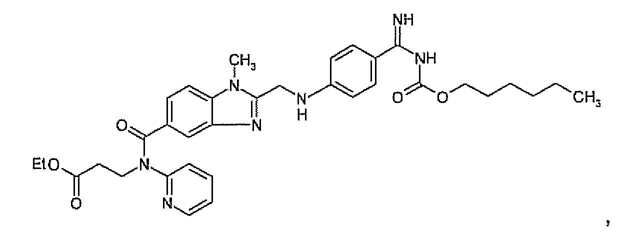 furthermore, Melagatran (D. Gustafsson, et al., The Direct Thrombin Inhibitor Melagatran and its Oral Prodrug H 376/95: Intestinal Absorption Properties, Biochemical and Pharmacodynamic Effects, Thromb. Res. 2001, Vol 101(3), 171-181) 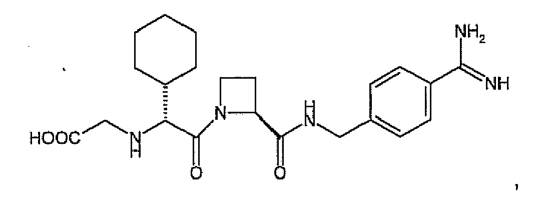 the prodrugs thereof, such as the orally active form Ximelagatran (H-376/95; J. I. Weitz, J. Hirsch; New Anticoagulant Drugs, Chest, 2001, Vol. 119, No.1 Suppl., 95S-107S) 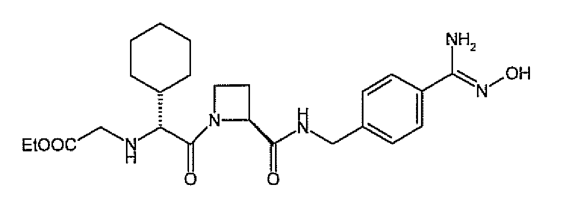 and the physiologically acceptable salts thereof. Prodrugs of the drugs mentioned above are such derivatives containing one or more groups capable of being cleaved in vivo, particularly a group which can be converted in-vivo into a carboxy group or/and a group capable of being cleaved in vivo from an imino or amino group. Compounds containing two groups capable of being cleaved in vivo are so-called double prodrugs. Groups which can be converted in-vivo into a carboxy group and groups capable of being cleaved in vivo from an imino or amino group are disclosed e.g. in WO 98/37075, being herewith incorporated by reference.

Thromboembolic disorders are meant to be such disorders or medical conditions being accompanied or characterized by elevated Thrombin formation or thrombin receptor expression or such conditions where elevated Thrombin plasma levels or elevated Thrombin receptor expression are involved or contribute in pathogenesis or progression of the disorder. This is the case for instance in disorders wherein elevated thrombin activity can lead to increased clot formation, thereby obstructing a venous or an arterial blood vessel at its site or by dislodgement and embolus formation in distant small and large vessels or lead to development of vascular syndromes, damages or diseases, atherosclerotic damages or arthritic conditions or stenosis by thrombin mediated vessel wall alterations such as proliferation and/or migration of vessel wall cells. Elevated thrombin activity are reported in connection with several thrombo-embolic disorders in the scientific literature regarding venous as well as arterial and microcirculatory disorders.

The indication "thromboembolic disorders" should be understood in a non-limiting manner to comprise
(a) vascular syndromes, damages or diseases,
   e.g. formation of either a plaque rupture and subsequent thromboembolic occlusion of a vessel such as in myocardial infarction or stroke or in the form of venous thrombosis leading to the risk of embolization and subsequent obstruction of major and minor arteries and venes;
(b) atherosclerotic damages,
   such as premature coronary atherosclerosis (Clin. Chem. Lab. 2001, 39(5): 380-4; Arterioscler. Thromb. Vasc. Biol. 2001, 21(9): 1446-50), stabilization of atherosclerotic plaques (Yonsei Med J 2000, 41(1): 82-8), particularly what is understood as plaques with thinned cap or plaques exposed to elevated levels of shear stress known to rupture easily (vulnerable plaque) and subsequently leading to massive activation of Thrombin and subsequent thrombus formation and potential embolization;
(c) proliferative and metastasizing diseases,
   such as cancer, e.g. mama carcinoma, cystic renal carcinomas (J. Urol. 2002, 168(1): 19-22), prostate cancer (Acta. Oncol. 2002, 41(3): 289-96), bladder cancer (J. Med. Invest. 2001, 48(1-2): 31-43), pancreatic carcinomas with liver metastasis, colon carcinomas with liver metastasis (J. Surg. Oncol. 2002, 80(2): 105-10, colorectal cancer (Br. J. Cancer 2002, 86(12): 1876-83), hepatocellular carcinoma (World J. Gastroenterol. 2002, 8(3): 385-92), ovarian carcinoma (Int. J. Oncol. 2000, 17(4): 673-81), including tumour invasion, metastasis and angiogenesis (Clin. Cancer Res. 2000 6(12): 4823-30; Pathol. Oncol. Res. 2001, 7(1):14-23);
(d) or, as further indication,
   the risk of thromboembolic diseases by endovascular procedures, intra-arterial or intravenous lines, implantation of devices, particularly those exposed to the blood flow, such as stents, valves, filters. etc, whereby this risk of thrombus formation is reduced by the method of the invention.

The method of prevention aspect of the invention applies especially to the indications of groups (a), (b) and (d).

According to the method of treatment and/or prevention according to the invention it is of advantage to maintain a plasma level of dipyridamole or mopidamole of about 0.2 to 5 µmol/L, preferably of about 0.4 to 5 µmol/L, especially of about 0.5 to 2 µmol/L or particularly of about 0.8 to 1.5 µmol/L. This can be achieved using any of the oral dipyridamole retard, instant or the parenteral formulations on the market, the retard formulations being preferred, for instance those available under the trademark Persantin^{®}, or, for the combination therapy with low-dose ASA, using those formulations available under the trademark Asasantin^{®} or Aggrenox^{®}. Dipyridamol retard formulations are also disclosed in EP-A-0032562, instant formulations are disclosed in EP-A-0068191 and combinations of ASA with dipyridamole are disclosed in EP-A-0257344 which are incorporated by reference. In case of mopidamole also oral retard, instant or a parenteral formulations can be used, e.g. those disclosed in GB 1,051,218 or EP-A-0,108,898 which are incorporated by reference, retard formulations being preferred.

Dipyridamole or mopidamole can be administered orally in a daily dosage of 25 to 1000 mg, preferably 50 to 900 mg, more preferred 100 to 480 mg, most preferred 150 to 400 mg. For long-term treatment it is of advantage to administer repeated doses such as a dose of 50 to 500 mg, preferably 200 to 400 mg of dipyridamole or mopidamole retard or any other instant release formulation three or four times a day. For parenteral administration dipyridamole or mopidamole could be given in a dosage of 0.5 to 5 mg/kg body weight, preferably 1 to 3.5 mg/kg body weight, during 24 hours as slow i.v. infusion (not faster than 0.2 mg/min).

As already mentioned hereinbefore dipyridamole, mopidamole or a pharmaceutically acceptable salt thereof can be used alone in a monopreparation or in combination with other antithrombotic agents, ACE inhibitors, Angiotensin II antagonists, Ca-antagonists or lipid-lowering agents for the treatment of MMP-9-dependent disorders.

Furthermore, the method of treatment and/or prevention according to the invention can be combined with any basic method of treatment or prevention known in the art for the above-identified disorders.

In case of atherosclerotic disorders this basic method of treatment or prevention may comprise administration of lipid-lowering agents such as HMG-Co-A reductase inhibitors or statins in the doses known in the art.

In case of arthritic conditions or inflammatory reactions this basic method of treatment or prevention may comprise administration of nonsteroidal anti-inflammatory drugs (NSAIDs) in the doses known in the art. Suitable NSAIDs for combination treatment are meant to include all COX (cyclooxygenase) inhibitors, e.g.
non-selective COX-inhibitors such as acetylsalicyclic acid, mesalazin,
ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenciofenac, alclofenac, bromfenac, ibufenac, aceclofenac, acemetacin, fentiazac, clidanac, etodolac, oxpinac,
mefenamic acid, meelofenamic acid, flufenamic acid, nifluminic acid, tolfenamic acid, diflunisal, flufenisal, piroxicam, tenoxicam, lornoxicam and nimesulide and the pharmaceutically acceptable salts thereof,
as well as selective COX 2-inhibitors such as meloxicam, celecoxib and rofecoxib and the pharmaceutically acceptable salts thereof.

In such combinations with any basic method of treatment or prevention known in the art each active ingredient can be administered either in accordance with its usual dosage range or a dose below its usual dosage range. The dosage for the combined NSAIDs or immunsuppressives is appropriately 1/50 of the lowest dose normally recommended up to 1/1 of the normally recommended dosage, preferably 1/20 to 1/2 and more preferably 1/10 to 1/5. The normally recommended dose for the combined drug should be understood to be the dose disclosed for example in Rote Liste^{®} 2002, Editio Cantor Verlag Aulendorf, Germany, or in Physician's Desk Reference.

In case of proliferative diseases this basic method of treatment or prevention may comprise administration of anti-tumour therapeutic agents, for topoisomerase inhibitors (e.g. etoposide), mitosis inhibitors (e.g. vinblastine), compounds which interact with nucleic acids (e.g. cis-platin, cyclophosphamide, adriamycin), hormone antagonists (e.g. tamoxifen), inhibitors of metabolic processes (e.g. 5-FU etc.), cytokines (e.g. interferons,or TNF-alpha) or antibodies, etc.

In case of conditions with elevated Thrombin receptor expression, the method of treatment according to the invention may be combined with drugs and substances which lead to elevated nitric oxide level (NO) such as from the group of statins. For combination treatment using dipyridamole or mopidamole together with statins any statin known in the art would be suitable, e.g. lovastatin, simvastatin, pravastatin, fluvastatin, atorvastatin or cerivastatin, using the dosages known in the art, for instance as described in Rote Liste^{®} 2002, Editio Cantor Verlag Aulendorf.

Dipyridamole or mopidamole in combination with low-dose ASA may be administered orally in a daily dosage of 10 to 30 mg of ASA together with 50 to 600 mg of dipyridamole or mopidamole, preferably 80 to 480 mg, most preferred 80 to 240 mg of dipyridamole or mopidamole, for instance in a weight ratio between 1 to 5 and 1 to 12, most preferred a weight ratio of 1 to 8, for instance 25 mg of ASA together with 200 mg of dipyridamole or mopidamole, typically given two times a day.

Other antithrombotic compounds would be given at 0.1 to 10 times, preferably at 0.3 to 5.0 times, most preferred at 0.3 to 2.0 times the clinically described dose (e.g. Rote Liste ^{®} 002, Editio Cantor Verlag Aulendorf; fradafiban, lefradafiban: EP-A-0483667), together with a daily dosage of 25 to 900 mg, preferably 50 to 480 mg, most preferred 75 to 400 mg of dipyridamole or mopidamole.

For prophylactic treatment heparin, low molecular weight heparin and heparinoids normally are given subcutaneously in one or more single (bolus) dosages units within 24 h. Therapeutically, these anticoagulants normally are given starting with an iv bolus, followed by a lower dose continuous iv administration. The dosages and timing of administration can be modified according to the needs of the patient, depending on coagulation analysis, severity of the underlying condition, side effects, responsiveness, age and weight of the patient.

For instance, heparin could be given prophylactically in a dosage range of 100 to 600 iU/kg bw 24 h (international units per kg body weight within 24 hours), preferably 200 to 300 iU/kg bw 24 h or specifically 300 iU/kg bw 24 h, together with a daily dosage of 25 to 900 mg, preferably 50 to 480 mg, most preferred 75 to 400 mg of dipyridamole or mopidamole, e.g. 80 to 240 mg of dipyridamole or mopidamole.

For instance, heparin could be given therapeutically in a dosage range of 300 to 800 iU/kg bw 24 h, preferably 400 to 500 iU/kg bw 24 h or specifically 500 iU/kg bw 24 h, together with a daily dosage of 25 to 900 mg, preferably 50 to 480 mg, most preferred 75 to 400 mg of dipyridamole or mopidamole, e.g. 80 to 240 mg of dipyridamole or mopidamole.

For instance, low molecular weight heparin could be given prophylactically in a dosage range of 5 to 25 mg/10 kg bw 24 h (milligram per 10 kg body weight within 24 hours), preferably 5 to 15 mg/10 kg bw 24 h or specifically 10 mg/10 kg bw 24 h, together with a daily dosage of 25 to 900 mg, preferably 50 to 480 mg, most preferred 75 to 400 mg of dipyridamole or mopidamole, e.g. 80 to 240 mg of dipyridamole or mopidamole. Normally, low molecular weight heparin is given in one or more single (bolus) dosage units of e.g. 5 mg/10 kg bw within 24 h.

For instance, low molecular weight heparin could be given therapeutically in a dosage of 10 to 50 mg/10 kg bw 24 h (milligram per 10 kg body weight within 24 hours), preferably 10 to 30 mg/10 kg bw 24 h or specifically 20 mg/10 kg bw 24 h, together with a daily dosage of 25 to 900 mg, preferably 50 to 480 mg, most preferred 75 to 400 mg of dipyridamole or mopidamole, e.g. 80 to 240 mg of dipyridamole or mopidamole. Normally, low molecular weight heparin is given in one or more single (bolus) dosage units of e.g. 5 mg/10 kg bw within 24 h.

For instance, heparinoids such as Orgaran^{®} could be given propylactically in a dosage range of 1500 to 4000 factor Xa units, preferably 1500 to 3000 factor Xa units, most preferred 1500 to 2500 factor Xa units or specifically 1500 or 2000 factor Xa units, together with a daily dosage of 25 to 900 mg, preferably 50 to 480 mg, most preferred 75 to 400 mg of dipyridamole or mopidamole, e.g. 80 to 240 mg of dipyridamole or mopidamole. Normally, heparinoids are given in one or more single (bolus) dosages of e.g. 500, 750 or 1250 factor Xa units within 24 h, preferably subcutaneously.

For instance, heparinoids such as Orgaran^{®} could be given therapeutically according to the following scheme:
1000 to 3000 factor Xa units as an iv bolus, continued by 400 units/h during 4 h, continued by 300 units/h during 4 h, continued by 150 to 200 units/h during 5 to 7 days. In parallel, the patient can be treated by parenteral administration of dipyridamole or mopidamole in a dosage of 0.5 to 5 mg/kg body weight, preferably 1 to 3.5 mg/kg body weight, during 24 hours as slow i.v. infusion (not faster than 0.2 mg/min).

For instance, warfarin could be given prophylactically in a dosage range of 0.1 to 5 INR (international normalized ratio) units, preferably 0.2 to 4.5 INR units, most preferred 0.5 to 3 INR units or specifically 2.5 INR units daily, together with a daily dosage of 25 to 900 mg, preferably 50 to 480 mg, most preferred 75 to 400 mg of dipyridamole or mopidamole, e.g. 80 to 240 mg of dipyridamole or mopidamole.

The thrombin-inhibitors, compound (A), (B), Melagatran or Ximelagatran, can be administered e.g. intravenously in a dosage of 0.01 to 3.0 mg/kg bw or, preferably, 0.03 to 1.0 mg/kg bw, furthermore orally in case of the prodrugs (compound (B) or Ximelagatran) of 0.3 to 30 mg/kg bw or, preferably, 0.1 to 10 mg/kg bw, one to four times a day.

For combination treatment using dipyridamole or mopidamole together with ACE inhibitors any ACE inhibitor known in the art would be suitable, e.g. benazepril, captopril, ceronapril, enalapril, fosinopril, imidapril, lisinopril, moexipril, quinapril, ramipril, trandolapril or perindopril, using the dosages known in the art, for instance as described in Rote Liste^{®} 2002, Editio Cantor Verlag Aulendorf.

For combination treatment using dipyridamole or mopidamole together with Angiotensin II antagonists any Angiotensin II antagonist known in the art would be suitable, e.g. the sartans such as candesartan, eprosartan, irbesartan, losartan, telmisartan, valsartan, olmesartan or tasosartan, using the dosages known in the art, for instance as described in Rote Liste^{®} 2002, Editio Cantor Verlag Aulendorf.

For combination treatment using dipyridamole or mopidamole together with Ca-antagonists any Ca-antagonist known in the art would be suitable, e.g. nifedipine, nitrendipine, nisoldipine, nilvadipine, isradipine, felodipine or lacidipine, using the dosages known in the art, for instance as described in Rote Liste^{®} 2002, Editio Cantor Verlag Aulendorf.

The pharmaceutical compositions according to the invention are meant to comprise a pyrimido-pyrimidine selected from dipyridamole, mopidamole and the pharmaceutically acceptable salts thereof, together with one or more drugs selected from the other antithrombotic agents, ACE inhibitors, Angiotensin II antagonists, Ca-antagonists and lipid-lowering agents mentioned under the method of treatment aspect, including all drugs mentioned specifically, and preferably adapted to be administered in the dosages mentioned hereinbefore. The pharmaceutical compositions according to the invention are meant to comprise a fixed dose combination comprising the active ingredients in one formulation together as well as a kit of parts comprising the active ingredients each in a separate containment, preferably in one package. The pharmaceutical composition may be adapted for simultaneous, separate or sequential administration.

For instance, a pharmaceutical composition according to the invention comprises a pyrimido-pyrimidine selected from dipyridamole, mopidamole and the pharmaceutically acceptable salts thereof, and a second active ingredient selected from warfarin, compound (A): 1-methyl-2-[N-(4-amidinophenyl)-aminomethyl]-benzimidazal-5-yl-carboxylic acid-N-(2-pyridyl)-N-(2-hydroxycarbonylethyl)-amide, and the prodrugs thereof, such as compound (B): dabigatran etexilate (1-ethyl-2-[N-[4-(N-n-hexyloxycarbonylamidino)phenyl]-aminomethyl]-benzimidazol-5-yl-carboxylic acid-N-(2-pyridyl)-N-(2-ethoxycarbanylethyl)-amide), Melagatran, the prodrugs thereof, such as Ximelagatran and the physiologically acceptable salts thereof, optionally together with one or more excipients or carriers.

With respect to all aspects of the invention mentioned hereinbefore dipyridamole and the pharmacologically acceptable salts thereof are preferred.

In order to study the inhibition of Thrombin receptor expression, patients surviving a stroke have been treated with conventional antiplatelet therapy (oral ASA) or a combination of Dipyridamole and ASA (Aggrenox) and thrombin receptor expression was measured during the course of treatment of one month. The expression of the complete as well as the cleaved Thrombin receptor was measured by flow cytometry using well established antibody technology on platelets after venipuncture. During the course of the treatment, a continuous and relevant decrease of complete as well as cleaved thrombin receptor on platelets in the Dipyridamole plus ASA treated group was found compared to the group treated with ASA alone.

### Example 1:

### Inhibition of Thrombin receptor expression in Platelets- by the Dipyridamole

**Table 1:**

| Parameter | Baseline (n=15) | 24 hr (n=15) | 3 days (n=15) | 7 days (n=15) | 15 days (n=14) | 30 days (n=12) |
|---|---|---|---|---|---|---|
| **ASA** | | | | | | |
| Thrombin receptor (PAR-1) SPAN 12 (intact receptor) | 34±7 | 37±7 | 28±9 | 24±5* | 26±4.* | 22±7* |
| Thrombin receptor (PAR-1) WEDE 15 (cleaved receptor) | 21.0±5.3 | 21.9±4.6 | 15.3±3.3* | 17.1±6.1 | 16.9±7.5 | 14.2±5.0* |

| **Aggrenox** | | | | | | |
|---|---|---|---|---|---|---|
| Thrombin receptor (PAR-1) SPAN 12 (intact receptor) | 38±6 | 24±6*† | 16±5*† | 19±4* | 15±4*† | 13±4*† |
| Thrombin receptor (PAR-1) WEDE 15 (cleaved receptor) | 22±3 | 12±5*† | 13±4* | 8±3*† | 9±3*† | 9±4.1*† |

| | | | | | | |
|---|---|---|---|---|---|---|
| • - represents significant (p<0.05) difference with own baseline; † - represents difference between groups | | | | | | |

Table 1 represents the mean and standard deviation of the relative numbers of expressed intact as well as cleaved Thrombin receptors on platelets over time after initiation of treatment with either ASA or the combination of Dipyridamole and ASA. Dipyridamole treatment shows a significant reduction of thrombin receptor on platelets.

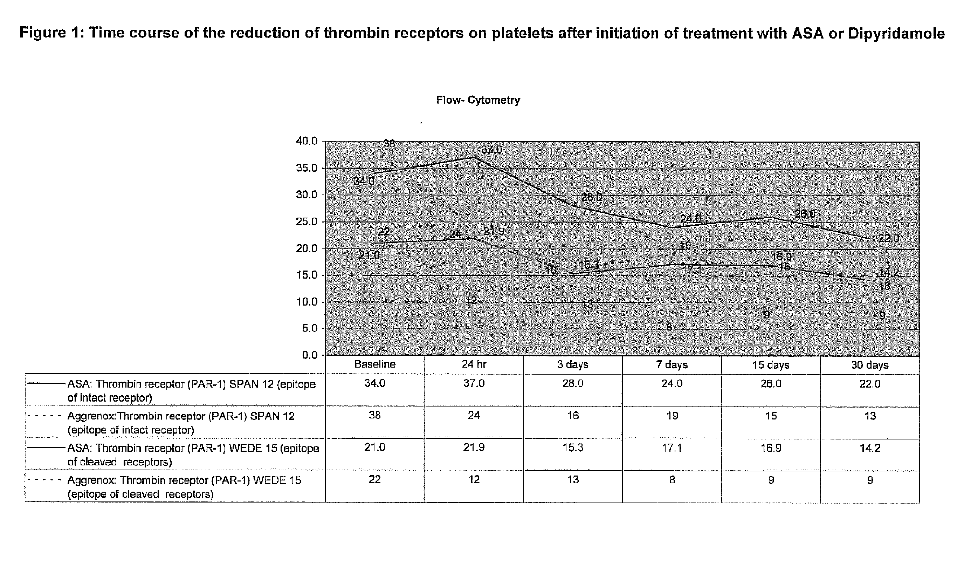

**Figure 1** **Legend:** Data for Thrombin receptor expression in patients undergoing therapy with ASA (solid lines) and Dipyridamole in combination with ASA (as in Aggrenox). During the time course of 30 days the treatment with ASA results in the known mild reduction of intact as well as cleaved Thrombin receptors on platelets ex vivo. The treatment with the combination of ASA plus Dipyridamole (as in Aggrenox) however significantly reduces the expression of Thrombin receptors on platelets particularly the cleaved Thrombin receptor indicating stabilization of platelets and control of thrombo-embolic processes.

## Claims

1. A method of treatment of the human or non-human animal body for treating or preventing disorders or of medical conditions related to elevated local or systemic elevation of thrombin or thrombin receptors such as thromboembolic or cardiovascular or cerebrovascular disease, said method comprising administering to said body an effective amount of a pharmaceutical composition comprising an active ingredient selected from dipyridamole, mopidamole and the pharmaceutically acceptable salts thereof, optionally in combination with one or more other antithrombotic agents or optionally in combination with an ACE inhibitor, Angiotensin II antagonist, Ca-antagonist or lipid-lowering agent particularly statins, antidiabetic agents including insulin.

2. The method of claim 1, **characterized in that** the disorder caused by elevated thrombin or thrombin receptors is selected from the group consisting of
(a) vascular syndromes, damages or diseases,
such as development of arterial or venous thrombosis, leading to myocardial infarction, stroke or other cardiovascular / cerebrovascular disorders or venous thrombosis;
(b) atherosclerotic damages,
such as premature coronary atherothrombosis or venous embolism;(c) proliferative diseases,
e.g. thrombin mediated embolic lodging of metastasis of metastasizing tumors;
(d) the risk of thromboembolic complications following the implantation of devices, in particular stents, valves, filters, intravenous or intra-arterial lines, or other devices giving raise to elevated thrombin or thrombin receptor formation / expression.

3. The method of claim 1 or 2, wherein a plasma level of the active ingredient of about 0.2 to 5 µmol/L is maintained.

4. The method of claim 1 or 2, wherein the active ingredient is administered orally in a daily dosage of 25 to 1000 mg or parenterally in a daily dosage of 0.5 to 5 mg/kg body weight.

5. The method of claim 1 or 2, wherein the active ingredient is administered alone in a monopreparation.

6. The method of claim 1 or 2, wherein the active ingredient is administered in combination with at least one other pharmaceutical active compound selected from the group consisting of an antithrombotic agent, an ACE inhibitor, an Angiotensin II antagonist, a Ca-antagonist, an antidiabetic agent including insulin, a lipid-lowering agent, and / or an anti-proliferative agent.

7. The method of claim 2, wherein the disorder caused by elevated thrombin or elevated thrombin receptor expression is an atherosclerotic disorder and the method comprises administration of a lipid-lowering agent.

8. The method of claim 2, wherein the disorder caused by elevated thrombin or elevated thrombin receptor expression is an arthritic condition or inflammatory reaction and the method comprises administration of a nonsteroidal anti-inflammatory drug (NSAID).

9. The method of claim 8, wherein the NSAID is selected from the group consisting of meloxicam, celecoxib, rofecoxib and the pharmaceutically acceptable salts thereof.

10. The method of claim 2, wherein the disorder caused by elevated thrombin or elevated thrombin receptor expression is a proliferative disease and the method comprises administration of an anti-tumour therapeutic agent.

11. The method of claim 1 or 2, wherein the active ingredient is administered orally in a daily dosage of 100 to 600 mg alone or in combination with 10 to 30 mg of ASA.

12. The use of a an active ingredient selected from dipyridamole, mopidamole and the pharmaceutically acceptable salts thereof, optionally in combination with one or more other antithrombotic agents or optionally in combination with an ACE inhibitor, Angiotensin II antagonist, Ca-antagonist or lipid lowering agent in particular from the group of statins, or a an antidiabetic agent including insulin, for the manufacture of a pharmaceutical composition for the treatment of the human or non-human animal body for treating or preventing disorders caused by elevated thrombin or elevated thrombin receptor expressionor of medical conditions.

13. The use of claim 12, wherein the disorder caused by elevated thrombin or elevated thrombin receptor expression is selected from the group consisting of
(a) vascular syndromes, damages or diseases,
such as development of arterial or venous thrombosis, leading to myocardial infarction, stroke or other cardiovascular / cerebrovascular disorders or venous thrombosis;
(b) atherosclerotic damages,
such as cerebrovascular or coronary atherothrombosis or venous embolism;(c) proliferative diseases,
e.g. thrombin mediated embolic lodging of metastasis of metastasising tumors;
(d) the risk of thromboembolic complications following the implantation of devices, in particular stents, valves, filters, intravenous or intra-arterial lines, or other devices giving raise to elevated thrombin formation or thrombin receptor expression.

14. The use of claim 12 or 13, wherein the pharmaceutical composition is a monopreparation comprising the active ingredient.

15. The use of claim 13 or 14, wherein the pharmaceutical composition comprises the active ingredient in combination with at least one other pharmaceutical active compound selected from the group consisting of an antithrombotic agent, an ACE inhibitor, an Angiotensin 11 antagonist, a Ca-antagonist, an antidiabetic agent including insulin and a lipid-lowering agent, in particular from the group of statins.

16. The use of claim 13, wherein the disorder caused by elevated thrombin or elevated thrombin receptor expression is an atherosclerotic disorder and the pharmaceutical composition comprises a lipid-lowering agent.

17. The use of claim 13, wherein the disorder caused by elevated thrombin or elevated thrombin receptor expression is an arthritic condition or inflammatory reaction and the pharmaceutical composition comprises a nonsteroidal anti-inflammatory drug (NSAID).

18. The use of claim 17, wherein the NSAID is selected from the group consisting of meloxicam, celecoxib, rofecoxib and the pharmaceutically acceptable salts thereof.

19. The use of claim 13, wherein the disorder caused by elevated thrombin or elevated thrombin receptor expressionis a proliferative disease and the pharmaceutical composition comprises an anti-tumour therapeutic agent.

20. The use of claim 12 or 13, wherein the pharmaceutical composition comprises an oral daily dosage unit of 50 to 600 mg of the active ingredient alone or in combination with an oral daily dosage unit of 10 to 30 mg of ASA.

21. A pharmaceutical composition comprising a pyrimido-pyrimidine selected from dipyridamole, mopidamole and the pharmaceutically acceptable salts thereof, and a second active ingredient selected from warfarin, compound (A): 1-methyl-2-[N-(4-amidinophenyl)-aminomethyl]-benzimidazol-5-yl-carboxylic acid-N-(2-pyridyl)-N-(2-hydroxycarbonylethyl)-amide, and the prodrugs thereof, such as compound (B): dabigatran etexilate (1-methyl-2-[N-[4-(N-n-hexyloxycarbonylamidino)phenyl]-aminomethyl]-benzimidazol-5-yl-carboxylic acid-N-(2-pyridyl)-N-(2-ethoxycarbonylethyl)-amide), Melagatran, the prodrugs thereof, such as Ximelagatran and the physiologically acceptable salts thereof, optionally together with one or more excipients or carriers.
